# EUROPÄISCHE PATENTSCHRIFT

(11) **EP 3 380 552 B1**
(45) Veröffentlichungstag und Bekanntmachung des Hinweises auf die Patenterteilung: **13.11.2019**
(21) Anmeldenummer: 16809298.9
(22) Anmeldetag: 23.11.2016
(51) Int. Cl.: C07C 407/00, C08J 3/22, C08K 3/22, C08K 5/01, C08K 5/10, C08K 5/14, C08K 5/12

(54) **BCHPC MIT VERRINGERTER ABBRANDGESCHWINDIGKEIT**
BCHPC HAVING A REDUCED BURNING RATE
BCHPC AYANT UNE VITESSE DE COMBUSTION RÉDUITE

(30) Priorität: 23.11.2015 DE 102015223051
(43) Veröffentlichungstag der Anmeldung: 03.10.2018
(73) Patentinhaber: United Initiators GmbH, 82049 Pullach (DE)
(72) Erfinder: NAGL, Iris, 81377 München (DE); DISCHL, Gudrun, 81373 München (DE); HERMANN, Dominik, 85221 Dachau (DE); CANELLA, Daniel, 81479 München (DE); KUNZ, Martin, 82544 Dettenhausen (DE); GUIGLEY, Kevin, Hernando Mississippi 38632 (US)
(74) Vertreter: Weickmann & Weickmann PartmbB
(86) Internationale Anmeldenummer: PCT/EP2016/078520
(87) Internationale Veröffentlichungsnummer: WO 2017/089375

(56) Entgegenhaltungen:
- EP-A1- 3 034 551
- WO-A1-01/46309
- DE-A1- 3 030 658
- DE-A1-102011 102 682

## Beschreibung

Gegenstand der vorliegenden Erfindung ist die Verringerung der Abbrandgeschwindigkeit von Di-(4-tert-butylcyclohexyl)-peroxycarbonat (BCHPC) durch Zugabe von Phlegmatisierungsmitteln sowie die Verwendung von BCHPC-Zubereitungen mit verringerter Abbrandgeschwindigkeit als Initiator in chemischen Reaktionen.

Di-(4-tert-butylcyclohexyl)-peroxycarbonat (BCHPC) ist ein vielgenutzter, fester Initiator zur Härtung von ungesättigten Polyesterharzen. Nachteilig an BCHPC ist seine relativ hohe Abbrandgeschwindigkeit, die dazu führt, dass dieses Peroxid in Gefahrklasse 1a (Deutschland) bzw. 1 (Niederlande und USA) eingeordnet ist (in Deutschland gemäß BG-Vorschrift B4, § 3 und Anhang 2, Stand Juni 2013). Damit sind hohe technische Anforderungen an eine sichere Lagerung verbunden.

Es war daher die Aufgabe der vorliegenden Erfindung, die Abbrandgeschwindigkeit von BCHPC zu senken und eine Zubereitung dieses Peroxycarbonats bereitzustellen, die eine gegenüber reinem BCHPC verringerte Abbrandgeschwindigkeit aufweist. Vorteilhafterweise sollte die Zubereitung sich ähnlich gut als Initiator in einer chemischen Reaktion eignen wie reines BCHPC.

Aus DE 1 618 726 und DE10 2011 102 682 A1 ist bekannt, dass sich Schlag- und Reibempfindlichkeit und die explosiven Eigenschaften organischer Peroxide verbessern lassen, wenn eine Zubereitung mit einem festen Phlegmatisierungsmittel hergestellt wird. Bisher unbekannt war, dass sich auf diese Weise auch die Abbrandgeschwindigkeit verringern lässt.

In der vorliegenden Erfindung wurde nun überraschend gefunden, dass die Abbrandgeschwindigkeit von BCHPC wesentlich verringert werden kann, wenn BCHPC mit bestimmten Substanzen vermischt wird, die dazu in der Lage sind, die Abbrandgeschwindigkeit zu verringern und dabei die gewünschte Funktionalität von BCHPC als Initiator für chemische Reaktionen aufrecht zu erhalten. Als zur Verringerung der Abbrandgeschwindigkeit geeignete Substanzen werden gemäß einem ersten Aspekt der Erfindung Phlegmatisierungsmittel verwendet. Der Begriff *"Phlegmatisierungsmittel"* bezeichnet im Sinne der Erfindung einen gegenüber BCHPC inerten Feststoff.

Ein erster Aspekt der vorliegenden Erfindung betrifft daher die Verwendung mindestens eines Phlegmatisierungsmittels zur Verringerung der Abbrandgeschwindigkeit von Di-(4-tert-butylcyclohexyl)-peroxycarbonat (BCHPC), umfassend das Bereitstellen eines Gemischs, welches BCHPC und mindestens ein Phlegmatisierungsmittel umfasst.

Als *"Phlegmatisierungsmittel"* im Sinne der vorliegenden Erfindung eignen sich grundsätzlich beliebige im Fachbereich bekannte Phlegmatisierungsmittel, wie sie beispielsweise in DE 1 6180726 und DE10 2011 102 682 A1 beschrieben werden. Besonders geeignet sind Dicyclohexylphtalat, Fluoren, Aluminiumtrihydroxid, Polystyrol, CC-Initiatoren wie CUROX CC-DC und Glycerintribenzoat sowie Kombinationen von zwei oder mehreren dieser Phlegmatisierungsmittel.

Das Phlegmatisierungsmittel wird in einer bevorzugten Ausführungsform in einer solchen Menge eingesetzt, dass das Verhältnis von BCHPC zu Phlegmatisierungsmittel 10:90 bis 90:10 Gew.-% beträgt. Bevorzugt sind 25:75 bis 75:25 Gew.-% oder 40:60 bis 60:40 Gew.-%.

Bei der Verwendung eines Phlegmatisierungsmittels zur Verringerung der Abbrandgeschwindigkeit von BCHPC werden vorzugsweise keine weiteren Komponenten zu dem Gemisch aus BCHPC und Phlegmatisierungsmittel zugegeben. Es ist jedoch grundsätzlich möglich, die Verwendung des Phlegmatisierungsmittels mit weiteren Zusatzstoffen zu kombinieren. Beispielsweise kann ein Gemisch von BCHPC und Phlegmatisierungsmittel weiterhin Polymere oder Füllstoffe umfassen. Beispiele für geeignete Füllstoffe sind Kreide, Silica, Silicate, Phtalate, und/oder Benzoate.

Der Anteil von BCHPC bezogen auf das Gesamtgemisch aus BCHPC und Phlegmatisierungsmittel sowie ggf. weiteren Bestandteilen beträgt in einer bevorzugten Ausführungsform 10 bis 90 Gew.-%, weiter bevorzugt 25 bis 75 Gew.-% oder 30 bis 60 Gew.-%.

Für die Verwendung eines oder mehrerer Phlegmatisierungsmittel zur Verringerung der Abbrandgeschwindigkeit von BCHPC können BCHPC und Phlegmatisierungsmittel in grundsätzlich beliebiger Weise miteinander kombiniert werden. Geeignete Methoden sind beispielsweise in DE 1 6180726 und DE10 2011 102 682 beschrieben.

In einem bevorzugten Aspekt der Erfindung umfasst die Verwendung eines oder mehrerer Phlegmatisierungsmittel zur Verringerung der Abbrandgeschwindigkeit von BCHPC die folgenden Schritte:
(i) Mischen von BCHPC mit mindestens einem Phlegmatisierungsmittel,
   wobei BCHPC in fester Form, in Form einer Lösung in einem organischen Lösungsmittel oder in Form einer wässrigen Suspension vorliegt und
   das mindestens eine Phlegmatisierungsmittel in fester Form, als Schmelze oder in Form einer Lösung oder Suspension in einem organischen oder wässrigen Lösungsmittel vorliegt,
(ii) ggf. Entfernen von Lösungsmittel und/oder Wasser und
(iii) ggf. Trocknen.

BCHPC wird vorzugsweise in fester Form, beispielsweise in Pulverform, eingesetzt. Dabei kann BCHPC trocken oder wasserfeucht vorliegen. Die Verwendung von BCHPC als trockenes Pulver ist bevorzugt. Alternativ kann BCHPC auch in Form einer wässrigen Suspension oder einer Lösung in einem organischen Lösungsmittel eingesetzt werden.

Das/die Phlegmatisierungsmittel wird/werden vorzugsweise ebenfalls in fester Form, beispielsweise in Pulverform, trocken oder wasserfeucht, verwendet. Alternativ ist auch eine Verwendung in Form einer Schmelze oder in Form einer Lösung oder Suspension in einem organischen oder wässrigen Lösungsmittel möglich.

Sofern BCHPC und/oder Phlegmatisierungsmittel in Form einer Lösung oder Suspension eingesetzt werden, erfolgt in Schritt (ii) eine Entfernung von Lösungsmittel und/oder Wasser. Hierfür eignen sich grundsätzlich beliebige Verfahrensschritte, wie beispielsweise eine Filtration. Das BCHPC-Phlegmatisierungsmittel-Gemisch wird dabei von der flüssigen Phase abgetrennt.

Abschließend kann in Schritt (iii) wahlweise eine Trocknung erfolgen. Alternativ kann auch auf eine Trocknung verzichtet werden und das BCHPC-Phlegmatisierungsmittel-Gemisch in wasserfeuchter Form bereitgestellt werden.

In einer weiteren Ausführungsform der Erfindung erfolgt die Verwendung von Phlegmatisierungsmittel zur Verringerung der Abbrandgeschwindigkeit von BCHPC bereits während der Herstellung von BCHPC. Hierfür kann mindestens ein Phlegmatisierungsmittel in einem Schritt (i) in einem Vorläufer von BCHPC oder in einer wässrigen Lösung oder Suspension des Vorläufers von BCHPC gelöst werden. Als Vorläufer von BCHPC kann beispielsweise Butylcyclohexylchlorformiat dienen. Die so erhaltene Lösung wird anschließend in Schritt (ii) mit einer Mischung aus wässriger Natriumperoxid- und Natriumhydroxidlösung umgesetzt. Das BCHPC/Phlegmatisierungsmittel-Gemisch wird in Schritt (iii) abgetrennt und abschließend in Schritt (iv) ggf. getrocknet.

Das aus der Verwendung eines oder mehrerer Phlegmatisierungsmittel zur Verringerung der Abbrandgeschwindigkeit von BCHPC resultierende Gemisch liegt in einer bevorzugten Ausführungsform der Erfindung in fester Form vor, beispielsweise in Pulverform. Dabei kann das Gemisch trocken oder in wasserfeuchter Form bereitgestellt werden.

In der vorliegenden Erfindung wurde weiterhin überraschend gefunden, dass sich die Abbrandgeschwindigkeit von BCHPC auch senken lässt, wenn mindestens ein weiteres organisches Peroxid zugemischt wird.

Ein weiterer Aspekt der vorliegenden Erfindung betrifft daher die Verwendung mindestens eines organischen Peroxids zur Verringerung der Abbrandgeschwindigkeit von BCHPC, umfassend das Bereitstellen eines Gemischs, welches BCHPC und mindestens ein weiteres organisches Peroxid umfasst. Dabei ist es besonders überraschend, dass die Initiatoraktivität von BCHPC aufrecht erhalten bleibt und in einigen Fällen sogar noch verbessert wird.

Für die Verwendung zur Verringerung der Abbrandgeschwindigkeit von BCHPC eignen sich erfindungsgemäß insbesondere organische Peroxide, die ausgewählt sind aus Dilauroylperoxid (LP), Dicetylperoxycarbonat (CEPC) und Dimyristylperoxycarbonat (MYPC). Ebenfalls geeignet sind Gemische von zwei oder mehreren dieser Peroxide.

Die Eignung von organischen Peroxiden zur Verringerung der Abbrandgeschwindigkeit von BCHPC ist besonders überraschend, da hierdurch weiterer Aktivsauerstoff in die Zubereitung eingebracht wird. Dennoch wurde gefunden, dass sich die Abbrandgeschwindigkeit wesentlich verringern lässt.

Das mindestens eine organische Peroxid wird in einer bevorzugten Ausführungsform in einer solchen Menge eingesetzt, dass das Verhältnis von BCHPC zu organischem Peroxid 10:90 bis 90:10 Gew.-% beträgt. Bevorzugt sind 25:75 bis 75:25 Gew.-% oder 40:60 bis 60:40 Gew.%.

Bei der Verwendung eines oder mehrerer organischer Peroxide zur Verringerung der Abbrandgeschwindigkeit von BCHPC werden vorzugsweise keine weiteren Komponenten zu dem Gemisch aus BCHPC und organischem Peroxid zugegeben. Es ist jedoch grundsätzlich möglich, die Verwendung von organischem Peroxid mit weiteren Zusatzstoffen zu kombinieren. Beispielsweise kann ein Gemisch von BCHPC und organischem Peroxid weiterhin Polymere oder Füllstoffe umfassen. Beispiele für geeignete Füllstoffe sind Kreide, Silica, Silicate, Phtalate, und/oder Benzoate.

Der Anteil von BCHPC bezogen auf das Gesamtgemisch aus BCHPC und organischem Peroxid sowie ggf. weiteren Bestandteilen beträgt in einer bevorzugten Ausführungsform 10 bis 90 Gew.-%, weiter bevorzugt 25 bis 75 Gew.-% oder 30 bis 60 Gew.-%.

Für die Verwendung eines oder mehrerer organischer Peroxide zur Verringerung der Abbrandgeschwindigkeit von BCHPC können BCHPC und organisches Peroxid in grundsätzlich beliebiger Weise miteinander kombiniert werden. Geeignete Methoden sind beispielsweise in DE 1 6180726 und DE10 2011 102 682 beschrieben.

In einer besonders bevorzugten Ausführungsform der vorliegenden Erfindung umfasst die Verwendung von mindestens einem organischen Peroxid zur Verringerung der Abbrandgeschwindigkeit von BCHPC die folgenden Schritte:
(i) Mischen von BCHPC mit mindestens einem weiteren organischen Peroxid, wobei BCHPC in fester Form, in Form einer Lösung in einem organischen Lösungsmittel oder in Form einer wässrigen Suspension vorliegt und
   das mindestens eine weitere organische Peroxid in fester Form, in Form einer Lösung in einem organischen Lösungsmittel oder in Form einer wässrigen Suspension vorliegt,
(ii) ggf. Entfernen von Lösungsmittel und/oder Wasser und
(iii) ggf. Trocknen.

BCHPC wird vorzugsweise in fester Form oder in Form einer wässrigen Suspension eingesetzt und mit einem oder mehreren organischen Peroxiden, die ebenfalls in fester Form oder in Form einer wässrigen Suspension vorliegen, vermischt. Besonders bevorzugt werden BCHPC und organisches Peroxid jeweils in fester, trockener oder wasserfeuchter Form eingesetzt und miteinander vermischt.

Sofern die Einzelkomponenten in Form einer Lösung oder Suspension eingesetzt werden, sieht Schritt (ii) die Entfernung von Lösungsmittel und/oder Wasser vor. Hier erfolgt eine Abtrennung des BCHPC/organischen Peroxid-Gemischs, beispielsweise durch Filtration.

Nach Bedarf kann in Schritt (iii) das erhaltene Gemisch getrocknet werden. Alternativ kann das Gemisch auch in wasserfeuchter Form bereitgestellt werden.

Das aus der Verwendung eines oder mehrerer organischer Peroxide zur Verringerung der Abbrandgeschwindigkeit von BCHPC resultierende Gemisch liegt in einer bevorzugten Ausführungsform der Erfindung in fester Form vor, beispielsweise in Pulverform. Dabei kann das Gemisch trocken oder in wasserfeuchter Form bereitgestellt werden.

In einer weiteren Ausführungsform der vorliegenden Erfindung wird eine Kombination von mindestens einem organischen Peroxid und mindestens einem Phlegmatisierungsmittel zur Verringerung der Abbrandgeschwindigkeit von BCHPC verwendet. Phlegmatisierungsmittel und organische Peroxide sind dabei wie vorstehend definiert.

Bei der Verwendung einer Kombination von organischem Peroxid und Phlegmatisierungsmittel zur Verringerung der Abbrandgeschwindigkeit von BCHPC werden vorzugsweise keine weiteren Komponenten zu dem Gemisch aus BCHPC, organischem Peroxid und Phlegmatisierungsmittel zugegeben. Es ist jedoch prinzipiell möglich, die Verwendung von organischem Peroxid und Phlegmatisierungsmittel mit weiteren Zusatzstoffen zu kombinieren. Beispielsweise kann ein BCHPC/organisches Peroxid/Phlegmatisierungsmittel-Gemisch weiterhin Polymere oder Füllstoffe umfassen. Beispiele für geeignete Füllstoffe sind Kreide, Silica, Silicate, Phtalate, und/oder Benzoate.

Die Gesamtmenge an organischem Peroxid und Phlegmatisierungsmittel wird vorzugsweise so gewählt, dass der Anteil an BCHPC bezogen auf das Gesamtgemisch 10 bis 90 Gew.-%, bevorzugt 25 bis 75 Gew.-% oder 30 bis 60 Gew.-% beträgt. Das Gemisch enthält bevorzugt keine zusätzlichen Bestandteile neben BCHPC, organischem Peroxid und Phlegmatisierungsmittel.

Bei der Kombination aus organischem Peroxid und Phlegmatisierungsmittel zur Verringerung der Abbrandgeschwindigkeit von BCHPC wird die Menge an organischem Peroxid vorzugsweise so gewählt, dass das Verhältnis von BCHPC zu weiterem organischen Peroxid 10:90 bis 90:10 Gew.-%, bevorzugt 25:75 bis 75:25 Gew.-% oder 40:60 bis 60:40 Gew.% beträgt. Die Menge an Phlegmatisierungsmittel wird vorzugsweise so gewählt, dass das Verhältnis von BCHPC zu Phlegmatisierungsmittel 10:90 bis 90:10 Gew.-%, bevorzugt 25:75 bis 75:25 Gew.-% oder 40:60 bis 60:40 Gew.% beträgt.

Für die Verwendung eines oder mehrerer Phlegmatisierungsmittel in Kombination mit einem oder mehreren organischen Peroxiden zur Verringerung der Abbrandgeschwindigkeit von BCHPC können die Einzelkomponenten in grundsätzlich beliebiger Weise miteinander kombiniert werden. Organisches Peroxid und Phlegmatisierungsmittel können gleichzeitig oder nacheinander mit BCHPC vermischt werden. Geeignete Methoden sind beispielsweise die in DE 1 618 726 und DE10 2011 102 682 für die Kombination von Peroxid und Phlegmatisierungsmittel beschriebenen Verfahren.

In einer bevorzugten Ausführungsform umfasst die Verwendung einer Kombination von organischem Peroxid und Phlegmatisierungsmittel zur Verringerung der Abbrandgeschwindigkeit von BCHPC die folgenden Schritte:
(i) Mischen von BCHPC mit dem mindestens einen organischen Peroxid und dem mindestens einen Phlegmatisierungsmittel,
   wobei BCHPC in fester Form, in Form einer Lösung in einem organischen Lösungsmittel oder in Form einer wässrigen Suspension vorliegt,
   das mindestens eine weitere organische Peroxid in fester Form, in Form einer Lösung in einem organischen Lösungsmittel oder in Form einer wässrigen Suspension vorliegt und
   das mindestens eine Phlegmatisierungsmittel in fester Form, in Form einer Lösung oder Suspension in einem organischen oder wässrigen Lösungsmittel vorliegt,
(ii) ggf. Entfernen von Lösungsmittel und/oder Wasser und
(iii) ggf. Trocknen.

Vorzugsweise werden BCHPC, Phlegmatisierungsmittel und organisches Peroxid jeweils in fester Form oder in Form einer wässrigen Suspension oder Lösung miteinander vermischt. Besonders bevorzugt liegen alle Bestandteile in fester Form, beispielsweise in Pulverform, trocken oder wasserfeucht, vor.

Sofern ein oder mehrere Bestandteile in Form einer Lösung oder Suspension eingesetzt werden, erfolgt in Schritt (ii) eine Abtrennung des BCHPC/organisches Peroxid/Phlegmatisierungsmittel-Gemischs von Lösungsmittel und/oder Wasser. Hierfür eignet sich beispielsweise eine Filtration.

Abschließend kann in Schritt (iii) das erhaltene Gemisch getrocknet werden. Alternativ kann das Gemisch auch in wasserfeuchter Form bereitgestellt werden.

Das aus der Verwendung einer Kombination aus organischem Peroxid und Phlegmatisierungsmittel zur Verringerung der Abbrandgeschwindigkeit von BCHPC resultierende Gemisch liegt in einer bevorzugten Ausführungsform der Erfindung in fester Form vor, beispielsweise in Pulverform. Dabei kann das Gemisch trocken oder in wasserfeuchter Form bereitgestellt werden.

Ein weiterer Aspekt der Erfindung betrifft eine Zusammensetzung umfassend BCHPC und mindestens eine Substanz zur Verringerung der Abbrandgeschwindigkeit, wobei die Substanz zur Verringerung der Abbrandgeschwindigkeit ausgewählt ist aus Dilaurylperoxid (LP), Diacetylperoxycarbonat (CEPC) und Dimyristylperoxycarbonat (MYPC) und Gemische davon. Die Substanz der Verringerung der Abbrandgeschwindigkeit ist ausgewählt aus organischen Peroxiden, Phlegmatisierungsmitteln und Gemischen davon, wie vorstehend definiert.

Die Zusammensetzung enthält vorzugsweise neben BCHPC, organischem Peroxid und Phlegmatisierungsmittel keine weiteren Bestandteile. Es ist aber grundsätzlich möglich, auch weitere Substanzen zuzugeben, wie insbesondere Polymere oder Füllstoffe. Beispiele für geeignete Füllstoffe sind Kreide, Silica, Silicate, Phtalate, und/oder Benzoate.

In einer bevorzugten Ausführungsform liegt die Zusammensetzung in fester Form vor, beispielsweise in Pulverform. Dabei kann die Zusammensetzung trocken oder in wasserfeuchter Form vorliegen.

Das Mengenverhältnis von BCHPC, organischem Peroxid und Phlegmatisierungsmittel kann nach Belieben eingestellt werden. Beispielsweise kann der Anteil an BCHPC bezogen auf die Zusammensetzung 10 bis 90 Gew.-%, bevorzugt 25 bis 75 Gew.-% oder 30 bis 60 Gew.-% betragen. In einer bevorzugten Ausführungsform enthält die Zusammensetzung neben BCHPC, organischem Peroxid und Phlegmatisierungsmittel keine weiteren Bestandteile.

In der erfindungsgemäßen Zusammensetzung liegt das Verhältnis von BCHPC zu weiterem organischen Peroxid vorzugsweise bei 10:90 bis 90:10 Gew.-%, weiter bevorzugt 25:75 bis 75:25 Gew.-% oder 40:60 bis 60:40 Gew.-%. Das Verhältnis von BCHPC zu Phlegmatisierungsmittel kann beispielsweise 10:90 bis 90:10 Gew.-%, bevorzugt 25:75 bis 75:25 Gew.-% oder 40:60 bis 60:40 Gew.-% betragen.

Eine erfindungsgemäße Zusammensetzung kann beispielsweise als Initiator in einer chemischen Reaktion eingesetzt werden. Besonders geeignet ist die Zusammensetzung zur Verwendung in einem Verfahren zur Härtung, beispielsweise eines ungesättigten Polyesterharzes.

Bei der Verwendung als Initiator für ein Verfahren zur Härtung erweist sich insbesondere der Einsatz eines oder mehrerer organischer Peroxide zur Verringerung der Abbrandgeschwindigkeit von BCHPC als besonders günstig. In diesem Fall gelingt es, die Härtungsgeschwindigkeit gegenüber unverdünntem BCHPC nahezu beizubehalten. Obwohl das Gemisch eine wesentlich geringere Abbrandgeschwindigkeit zeigt, bleibt die Härtungsgeschwindigkeit bei einer Verwendung als Initiator zur Härtung eines ungesättigten Polyesterharzes im Wesentlichen unbeeinflusst. Als besonders geeignet erweist sich in diesem Zusammenhang die Verwendung von Dicetylperoxycarbonat (CEPC). Hier wurde überraschend gefunden, dass die Härtungsgeschwindigkeit gegenüber unverdünntem BCHPC sogar zunimmt.

Die Erfindung soll durch die folgenden Figuren und Beispiele noch weiter veranschaulicht werden.
- **Figur 1:**: Abbrandgeschwindigkeit von BCHPC im Vergleich zu verschiedenen BCHPC-Zubereitungen mit Phlegmatisierungsmitteln. Gezeigt sind Zubereitungen von BCHPC mit CUROX LP-S, Glycerintribenzoat, CUROX CC-DC, Aluminiumtrihydroxid, Polystyrol, Dicyclohexylphthalat oder Fluoren.
- **Figur 2:**: Abbrandgeschwindigkeit von BCHPC mit Dicyclohexylphthalat in unterschiedlichen Mischungsverhältnissen.
- **Figur 3:**: Abbrandgeschwindigkeiten von BCHPC-Zubereitungen mit verschiedenen organischen Peroxiden.
Gezeigt sind in **Figur 3A** Zubereitungen von BCHPC mit CUROX LP-S in verschiedenen Mischungsverhältnissen im Vergleich zu reinem CUROX LP-S und BCHPC.
In **Figur 3B** sind die Ergebnisse für Mischungen von BCHPC mit CEPC 65% in verschiedenen Mischungsverhältnissen und für Mischungen von BCHPC mit MYPC oder mit einer Kombination aus CUROX LP-S und MYPC im Vergleich zu reinem BCHPC gezeigt.
- **Figur 4:**: Temperaturverlauf bei der Härtung mit verschiedenen BCHPC-Zubereitungen. Gezeigt sind die Ergebnisse für trockenes BCHPC (BCHPC), wasserfeuchtes BCHPC (BCHPC-75-W), trockenes CEPC (CEPC), wasserfeuchtes CEPC 65% und für Kombinationen von trockenem BCHPC mit CUROX LP-S, CEPC 65% oder CUROX LP-S und MYPC.

### Beispiele

### 1. Untersuchung der Abbrandgeschwindigkeit

### 1.1 Kombination von BCHPC mit verschiedenen Phlegmatisierungsmitteln

Es wurden verschiedene Mischungen von BCHPC mit unterschiedlichen Phlegmatisierungsmitteln hergestellt und auf ihre Abbrandgeschwindigkeiten untersucht.

Getestet wurden die folgenden Kombinationen von BCHPC mit Peroxid oder Phlegmatisierungsmitteln:
BCHPC : CUROX LP-S(2 : 1)
BCHPC : Glycerintribenzoat (2 : 1)
BCHPC : CUROX CC-DC (2 : 1)
BCHPC : Aluminiumtrihydroxid (2 : 1)
BCHPC : Polystryol (2 : 1)
BCHPC : Dicyclohexylphthalat (2 : 1)
BCHPC : Fluoren (2:1)
BCHPC

Die Angaben der Mengenverhältnisse beschreiben jeweils Gewichtsverhältnisse.

CUROX LP-S ist ein 75 bis 80%iges, wasserfeuchtes Dilauroylperoxid von United Initiators GmbH & Co. KG.

CUROX CC-DC (bzw. CCDFB-90) ist ein nichtperoxidischer CC-Initiator von United Initiators GmbH & Co. KG, 2,3-Dimethyl-2,3-diphenybutan.

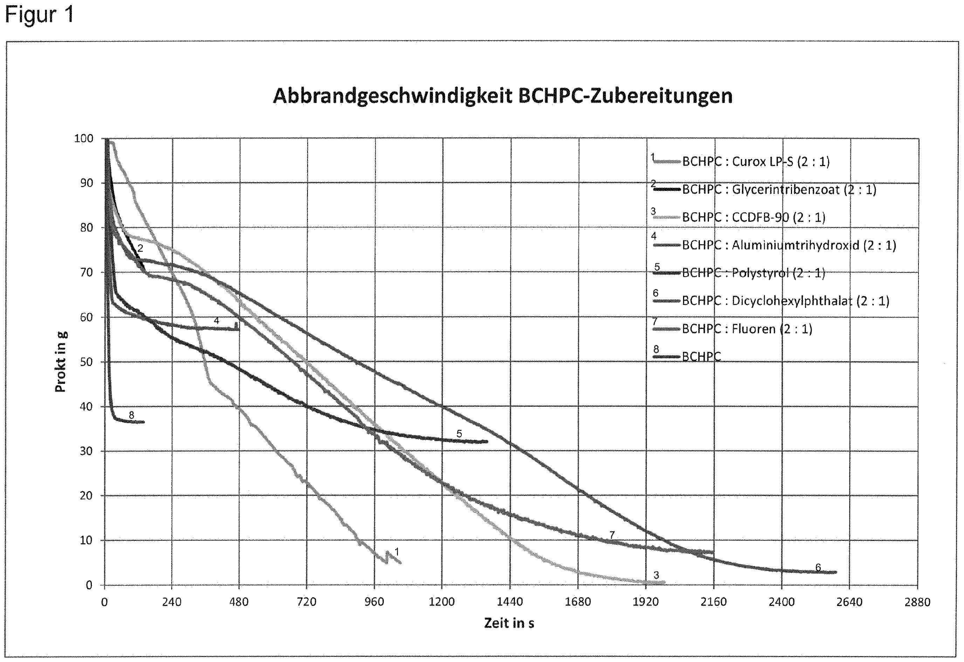

Um die Abbrandgeschwindigkeiten der verschiedenen Mischungen vergleichen zu können, wurden jeweils 100 g der Zubereitung entzündet und die noch verbliebene Menge der Substanz gegenüber der Zeit bestimmt. Das Vorgehen entspricht BG-Vorschrift B4, Anhang 4, Fassung vom 01.01.1997, Anhang 4 für Flüssigkeiten. Das Vorgehen wurde auf Feststoffe angepasst. Die Ergebnisse sind in Figur 1 gezeigt.

Es wurde gefunden, dass die Abbrandgeschwindigkeit sinkt, wenn BCHPC-Pulver mit einem inerten Feststoff, wie Dicyclohexylphthalat, Fluoren, Aluminiumtrihydroxid, Polystyrol und Glycerintribenzoat, vermischt wird.

### 1.2 Kombination von BCHPC mit Dicylohexylphthalat in unterschiedlichen Mischungsverhältnissen

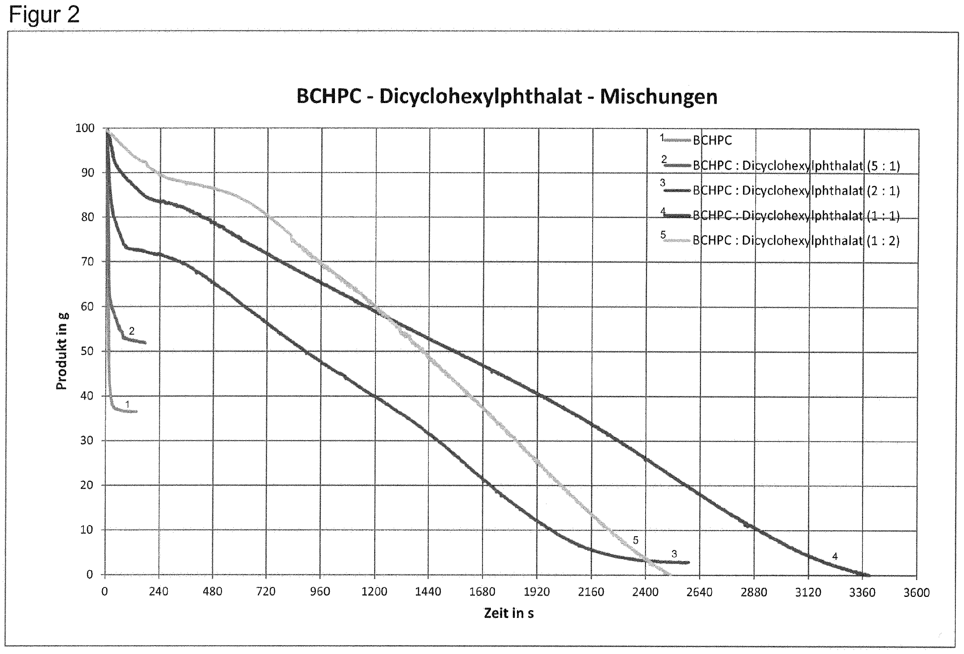

Um den Einfluss der Menge des verwendeten Phlegmatisierungsmittels auf die Abbrandgeschwindigkeit zu untersuchen, wurden Kombinationen von BCHPC mit Dicyclohexylphthalat in unterschiedlichen Mischungsverhältnissen hergestellt. Die Abbrandgeschwindigkeit wurde wie in Beispiel 1.1 beschrieben untersucht. Die getesteten Mischungen waren BCHPC : Dicyclohexylphthalat im Verhältnis 5:1, 2:1 und 1:1 sowie reines BCHPC. Dabei zeigte sich, dass bei Zusatz einer größeren Menge an Phlegmatisierungsmittel auch die Abbrandgeschwindigkeit weiter sinkt. Die Ergebnisse sind in Figur 2 gezeigt.

### 1.3 Kombination von BCHPC mit organischem Peroxid

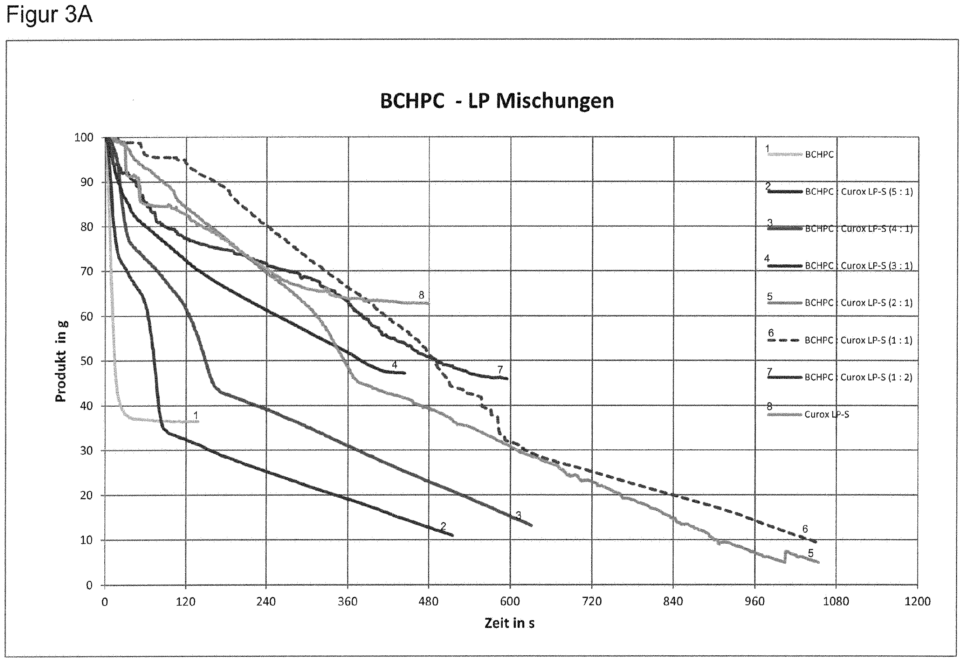

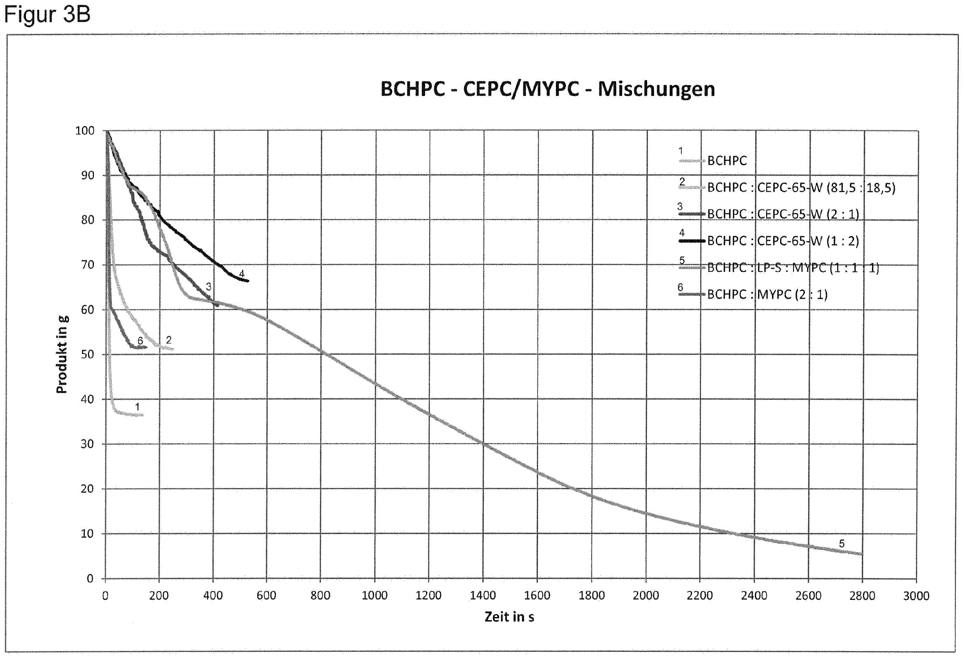

Um die Abbrandgeschwindigkeit eines Gemischs von BCHPC mit einem oder mehreren weiteren organischen Peroxiden zu untersuchen, wurden Kombinationen von BCHPC mit Dilauroylperoxid (LP) bzw. Dicetylperoxycarbonat (CEPC) in verschiedenen Mischungsverhältnissen hergestellt. Außerdem wurden Kombinationen von BCHPC mit Dilauroylperoxid und Myristylperoxycarbonat (MYPC) im Verhältnis 1:1:1 bzw. BCHPC mit MYPC im Verhältnis 2:1 untersucht. Die Untersuchung der Abbrandgeschwindigkeit wurde wie in Beispiel 1.1 beschrieben durchgeführt. Die Ergebnisse sind in den Figuren 3A und 3B gezeigt.

Für Mischungen von BCHPC und LP wurden Vergleiche von Mischungsverhältnissen von 5:1, 4:1, 3:1, 2:1, 1:1 und 1:2 untersucht. Die Abbrandgeschwindigkeit war in jedem Fall geringer, als für reines BCHPC beobachtet. Bei einem Überschuss von Dilauroylperoxid (BCHP : CUROX LP-S = 1:2) ist die Abbrandgeschwindigkeit zwar nicht so deutlich verringert wie bei einem Verhältnis von 1:1, das Entzünden der Mischung dauerte jedoch deutlich länger als bei den anderen Mischungen.

Für Kombinationen von BCHPC mit Dicetylperoxycarbonat wurden die Mischungsverhältnisse 81,5:18,5, 2:1 und 1:2 untersucht.

Es ist erkennbar, dass sich in allen Fällen der Abbrand der Mischungen gegenüber reinem BCHPC erheblich verzögert, obwohl das BCHPC mit einem bzw. mehreren weiteren organischen Peroxiden kombiniert wird, durch welche weiterer Aktivsauerstoff in die Zubereitung eingebracht wird.

### 2. Härtungsgeschwindigkeit verschiedener BCHPC-Zubereitungen

Um die Härtungsgeschwindigkeit von Mischungen aus BCHPC und verschiedenen organischen Peroxiden zu untersuchen, wurde ein Härtungstest gemäß DIN16945 durchgeführt. Untersucht wurden die folgenden Mischungen:
- BCHPC
- BCHPC-75-W
- BCHPC/CUROX LP-S (80 %) - 2:1
- BCHPC /CEPC 65% - 2:1
- BCHPC /CEPC 65% (81,5:18,5)
- CEPC 65%
- CEPC
- BCHPC/CUROX LP-S/MYPC (1:1:1)

CEPC 65% ist ein ca. 65%iges wasserfeuchtes Dicetylperoxydicarbonat.

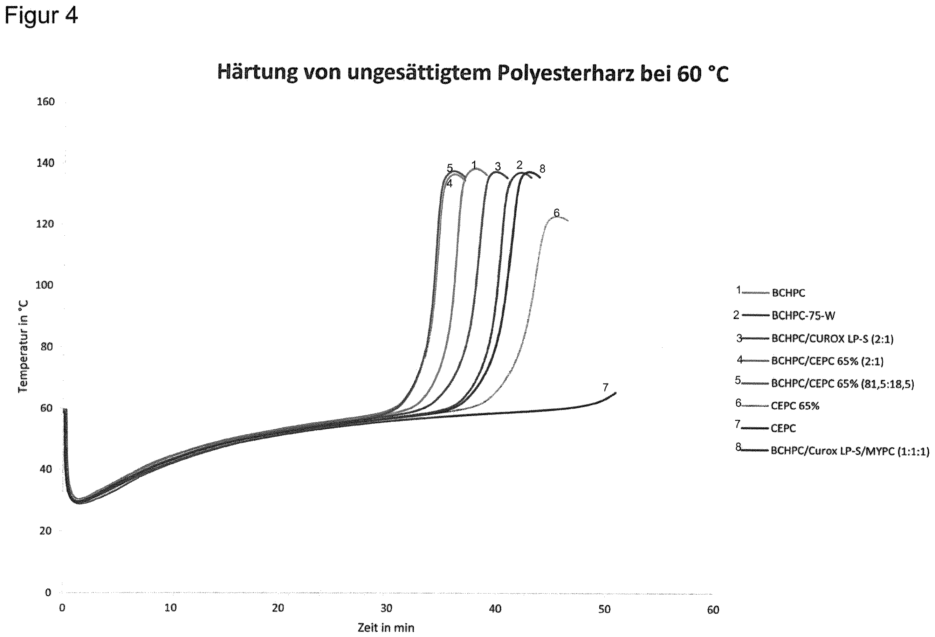

Es zeigte sich, dass bei den verschiedenen untersuchten Mischungen die Härtungsgeschwindigkeit gegenüber unverdünntem BCHPC nur wenig abnimmt. Im Fall der Mischung aus BCHPC und wasserfeuchtem CEPC-Pulver wurde sogar eine Zunahme der Härtungsgeschwindigkeit beobachtet. Die Ergebnisse sind in Figur 4 gezeigt.

## Patentansprüche

1. Verwendung mindestens eines organischen Peroxids zur Verringerung der Abbrandgeschwindigkeit von Di-(4-tert-butylcyclohexyl)-peroxycarbonat (BCHPC), umfassend
Bereitstellen eines Gemischs, welches BCHPC und mindestens ein weiteres organisches Peroxid und ggf. mindestens ein Phlegmatisierungsmittel umfasst, wobei das mindestens eine Phlegmatisierungsmittel ein gegenüber BCHPC inerter Feststoff ist.

2. Verwendung nach Anspruch 1, wobei das Gemisch in fester Form vorliegt, vorzugsweise in Pulverform.

3. Verwendung nach Anspruch 1 oder 2, wobei mindestens ein organisches Peroxid verwendet wird, das ausgewählt ist aus Dilauroylperoxid (LP), Dicetylperoxycarbonat (CEPC) und Dimyristylperoxycarbonat (MYPC).

4. Verwendung nach einem der vorhergehenden Ansprüche, wobei mindestens ein Phlegmatisierungsmittel verwendet wird, das ausgewählt ist aus Dicyclohexylphthalat, Fluoren, Aluminiumtrihydroxid, Polystyrol, und Glycerintribenzoat.

5. Verwendung nach einem der vorhergehenden Ansprüche, wobei der Anteil an BCHPC bezogen auf das Gemisch aus BCHPC und weiterem organischem Peroxid und ggf. Phlegmatisierungsmittel 10 bis 90 Gew.-%, bevorzugt 25 bis 75 Gew.-% oder 30 bis 60 Gew.-% beträgt.

6. Verwendung nach einem der vorhergehenden Ansprüche, wobei das Verhältnis von BCHPC zu weiterem organischen Peroxid 10:90 bis 90:10 Gew.-%, bevorzugt 25:75 bis 75:25 Gew.-% oder 40:60 bis 60:40 Gew.-% beträgt.

7. Verwendung nach einem der vorhergehenden Ansprüche, wobei das Verhältnis von BCHPC zu Phlegmatisierungsmittel 10:90 bis 90:10 Gew.-%, bevorzugt 25:75 bis 75:25 Gew.-% oder 40:60 bis 60:40 Gew.-% beträgt.

8. Verwendung nach einem der vorhergehenden Ansprüche, wobei das Gemisch weiterhin Polymere oder anorganische oder organische Füllstoffe umfasst.

9. Verwendung nach einem der vorhergehenden Ansprüche, umfassend die Schritte
(i) Mischen von BCHPC mit dem mindestens einen weiteren organischen Peroxid,
wobei BCHPC in fester Form, in Form einer Lösung in einem organischen Lösungsmittel oder in Form einer wässrigen Suspension vorliegt und
das mindestens eine weitere organische Peroxid in fester Form, in Form einer Lösung in einem organischen Lösungsmittel oder in Form einer wässrigen Suspension vorliegt,
(ii) ggf. Entfernen von Lösungsmittel und/oder Wasser und
(iii) ggf. Trocknen.

10. Verwendung nach Anspruch 1, wobei das mindestens eine organische Peroxid in fester Form oder in Form einer wässrigen Suspension, mit BCHPC in fester Form oder in Form einer wässrigen Suspension vermischt wird und anschließend ggf. Wasser entfernt wird.

11. Verwendung nach einem der vorhergehenden Ansprüche, wobei das mindestens eine weitere organische Peroxid und das BCHPC in trockener oder wasserfeuchter fester Form vermischt werden.

12. Zusammensetzung, umfassend BCHPC und mindestens eine Substanz zur Verringerung der Abbrandgeschwindigkeit, wobei die Substanz zur Verringerung der Abbrandgeschwindigkeit ausgewählt ist aus Dilauroylperoxid (LP), Dicetylperoxycarbonat (CEPC) und Dimyristylperoxycarbonat (MYPC)und Gemischen davon.

13. Zusammensetzung nach Anspruch 12, welche weiterhin mindestens ein Phlegmatisierungsmittel umfasst, welches ein gegenüber BCHPC inerter Feststoff ist, vorzugsweise ausgewählt aus Dicyclohexylphthalat, Fluoren, Aluminiumtrihydroxid, Polystyrol, und Glycerintribenzoat.

14. Verwendung einer Zusammensetzung nach Anspruch 12 oder 13, als Initiator in einer chemischen Reaktion, insbesondere in einem Verfahren zur Härtung z.B. eines ungesättigten Polyesterharzes.

## Claims

1. Use of at least one organic peroxide for reducing the burn rate of di-(4-tert-butylcyclohexyl)-peroxycarbonate (BCHPC), which comprises providing a mixture comprising BCHPC and at least one further organic peroxide and optionally at least one desensitising agent, wherein the at least one desensitising agent is a solid that is inert with respect to BCHPC.

2. Use according to claim 1, wherein the mixture is in solid form, preferably in powder form.

3. Use according to either claim 1 or claim 2, wherein at least one organic peroxide is used which is selected from dilauroyl peroxide (LP), dicetyl peroxycarbonate (CEPC) and dimyristyl peroxycarbonate (MYPC).

4. Use according to any of the preceding claims, wherein at least one desensitising agent is used which is selected from dicyclohexyl phthalate, fluorene, aluminium trihydroxide, polystyrene, and glycerol tribenzoate.

5. Use according to any of the preceding claims, wherein the proportion of BCHPC, based on the mixture of BCHPC and further organic peroxide and optionally a desensitising agent, is 10 to 90 wt.%, preferably 25 to 75 wt.% or 30 to 60 wt.%.

6. Use according to any of the preceding claims, wherein the ratio of BCHPC to further organic peroxide is 10:90 to 90:10 wt.%, preferably 25:75 to 75:25 wt.% or 40:60 to 60:40 wt.%.

7. Use according to any of the preceding claims, wherein the ratio of BCHPC to desensitising agent is 10:90 to 90:10 wt.%, preferably 25:75 to 75:25 wt.% or 40:60 to 60:40 wt.%.

8. Use according to any of the preceding claims, wherein the mixture further comprises polymers or inorganic or organic fillers.

9. Use according to any of the preceding claims, comprising the steps of
(i) mixing BCHPC with the at least one further organic peroxide, wherein BCHPC is in solid form, in the form of a solution in an organic solvent or in the form of an aqueous suspension, and the at least one further organic peroxide is in solid form, in the form of a solution in an organic solvent or in the form of an aqueous suspension,
(ii) optionally removing solvent and/or water and
(iii) optionally drying.

10. Use according to claim 1, wherein the at least one organic peroxide in solid form or in the form of an aqueous suspension is mixed with BCHPC in solid form or in the form of an aqueous suspension, and then water is optionally removed.

11. Use according to any of the preceding claims, wherein the at least one further organic peroxide and the BCHPC are mixed in dry or water-moist solid form.

12. Composition comprising BCHPC and at least one substance for reducing the burn rate, wherein the substance for reducing the burn rate is selected from dilauroyl peroxide (LP), dicetyl peroxycarbonate (CEPC) and dimyristyl peroxycarbonate (MYPC) and mixtures thereof.

13. Composition according to claim 12, further comprising at least one desensitising agent which is a solid that is inert with respect to BCHPC and is preferably selected from dicyclohexyl phthalate, fluorene, aluminium trihydroxide, polystyrene, and glycerol tribenzoate.

14. Use of a composition according to either claim 12 or claim 13 as an initiator in a chemical reaction, in particular in a method for curing e.g. an unsaturated polyester resin.

## Revendications

1. Utilisation d'au moins un peroxyde organique pour la réduction de la vitesse de combustion du di-(4-tert-butylcyclohexyl)-peroxycarbonate (BCHPC), comprenant la fourniture d'un mélange qui comprend du BCHPC et au moins un autre peroxyde organique, et le cas échéant, au moins un flegmatisant, dans laquelle l'au moins un flegmatisant est un solide inerte vis-à-vis du BCHPC.

2. Utilisation selon la revendication 1, dans laquelle le mélange se présente sous forme solide, de préférence sous forme pulvérulente.

3. Utilisation selon la revendication 1 ou 2, dans laquelle au moins un peroxyde organique est utilisé, qui est choisi parmi le dilauroylperoxyde (LP), le dicétylperoxycarbonate (CEPC), et le dimyristylperoxycarbonate (MYPC).

4. Utilisation selon l'une des revendications précédentes, dans laquelle au moins un flegmatisant est utilisé, qui est choisi parmi le dicyclohexylphtalate, le fluorène, le trihydroxyde d'aluminium, le polystyrène et le tribenzoate de glycérine.

5. Utilisation selon l'une des revendications précédentes, dans laquelle la proportion de BCHPC par rapport au mélange de BCHPC et de l'autre peroxyde organique, et le cas échéant, du flegmatisant est de 10 à 90 % en poids, de préférence de 25 à 75 % en poids ou de 30 à 60 % en poids.

6. Utilisation selon l'une des revendications précédentes, dans laquelle le rapport du BCHPC à l'autre peroxyde organique est de 10:90 à 90:10 % en poids, de préférence de 25:75 à 75:25 % en poids ou de 40:60 à 60:40 % en poids.

7. Utilisation selon l'une des revendications précédentes, dans laquelle le rapport du BCHPC sur le flegmatisant est de 10:90 à 90:10 % en poids, de préférence de 25:75 à 75:25 % en poids ou de 40:60 à 60:40 % en poids.

8. Utilisation selon l'une des revendications précédentes, dans laquelle le mélange comprend en outre des polymères ou des charges inorganiques ou organiques.

9. Utilisation selon l'une des revendications précédentes, comprenant les étapes consistant à
(i) mélanger du BCHPC avec l'au moins un autre peroxyde organique,
dans laquelle le BCHPC se présente sous forme solide, sous la forme d'une solution dans un solvant organique ou sous la forme d'une suspension aqueuse, et
l'au moins un autre peroxyde organique se présente sous forme solide, sous la forme d'une solution dans un solvant organique ou sous la forme d'une suspension aqueuse,
(ii) le cas échéant éliminer le solvant et/ou l'eau et
(iii) le cas échéant sécher.

10. Utilisation selon la revendication 1, dans laquelle l'au moins un peroxyde organique est mélangé sous forme solide ou sous la forme d'une suspension aqueuse avec le BCHPC sous forme solide ou sous la forme d'une suspension aqueuse, et l'eau est ensuite éliminée le cas échéant.

11. Utilisation selon l'une des revendications précédentes, dans laquelle l'au moins un autre peroxyde organique et le BCHPC sont mélangés sous une forme sèche ou solide humidifiée avec de l'eau.

12. Composition comprenant du BCHPC et au moins une substance pour la réduction de la vitesse de combustion, dans laquelle la substance pour la réduction de la vitesse de combustion est choisie parmi le dilauroylperoxyde (LP), le dicétylperoxycarbonate (CEPC) et le dimyristylperoxycarbonate (MYPC) et leurs mélanges.

13. Composition selon la revendication 12, qui comprend en outre au moins un flegmatisant qui est un solide inerte vis-à-vis du BCHPC, de préférence choisi parmi le dicyclohexylphtalate, le fluorène, le trihydroxyde d'aluminium, le polystyrène et le tribenzoate de glycérine.

14. Utilisation d'une composition selon la revendication 12 ou 13, en tant qu'initiateur dans une réaction chimique, en particulier dans un procédé pour le durcissement par exemple d'une résine de polyester insaturée.
